(19) Europäisches Patentamt
European Patent Office
Office européen des brevets

(11) Publication number: **0 444 691 A1**

(12) # EUROPEAN PATENT APPLICATION

(21) Application number: **91103057.5**

(22) Date of filing: **01.03.91**

(51) Int. Cl.5: **A61K 39/39**, A61K 45/05

(30) Priority: **02.03.90 US 487438**

(43) Date of publication of application:
**04.09.91 Bulletin 91/36**

(84) Designated Contracting States:
**AT BE CH DE DK ES FR GB GR IT LI LU NL SE**

(71) Applicant: **DU PONT MERCK PHARMACEUTICAL COMPANY**
**Wilmington, Delaware 19880-0400(US)**

(72) Inventor: **Neville, Mary Elizabeth**
**143 Garvin Boulevard**
**Sharon Hill, Pennsylvania 19079(US)**

(74) Representative: **von Kreisler, Alek, Dipl.-Chem. et al**
**Patentanwälte Von Kreisler-Selting-Werner,**
**Deichmannhaus am Hauptbahnhof**
**W-5000 Köln 1(DE)**

(54) **Use of interleukin-1 to enhance the immune response of weakly immunogenic tumors.**

(57) There is described the use of IL-1 to enhance or induce an immune response to weakly or non-immunogenic tumors. Further described is a method of treating a weakly or non-immunogenic tumor in a mammal by treatment with IL-1 prior to cyto-reductive therapy such as radiation therapy or surgery.

EP 0 444 691 A1

## FIELD OF THE INVENTION

This invention relates to the use of interleukin-1 (IL-1) to enhance the immune response to weakly or non-immunogenic tumors.

## BACKGROUND OF THE INVENTION

IL-1 is a protein secreted by macrophages, monocytes and other cells when activated by appropriate stimuli (Oppenheim et al. (1986) Immunology Today 7:45), IL-1 has many diverse biological functions including the induction of fever and neutrophilia, stimulation of acute-phase protein synthesis, and procoagulant activity by endothelial cells. IL-1$\beta$ has also been shown to enhance T and B cell immune responses in vitro (Hoffmann (1979) Ann N.Y. Acad. Sci. 322:577; Wood (1979) J. Immunology 123:2400; Smith (1980) Immunol. Rev. 51:337) and in vivo (Staruch and Wood, (1983) J. Immunology 130:2191; Reed et al. (1989) J. Immunology 142:3129; North et al. (1988) J. Exp. Med 168:2031; Reed et al. (1989) J. Immunology 142:2067). In vivo IL-1 has been shown to act like an adjuvant. An adjuvant is a substance which non-specifically enhances the immune response to an antigen (Klein (1982) Immunology: The Science Of Self-Nonself). Injections of mice with IL-1 increases the in vitro primary and secondary antibody responses to sheep red blood cells (SRBC) and to tri-nitrophenyl (TNP)-haptens (Staruch and Wood (1983) J. Immunology 130:2191) and the secondary response to proteins (Reed et al. (1989) J. Immunology 142:3129). IL-1 has also been reported to enhance T cell responses to immunogenic tumors in mice (North et al. (1988) J. Exp. Med 168:2031), but not to non-immunogenic tumors (Nakamura et al. (1986) GANN 77:767; Nakata et al. (1988) Cancer Res. 48:584). North et al. reported that IL-1$\beta$ caused complete regression of immunogenic tumors, but did not cause regression of weakly or poorly immunogenic tumors (North et al. (1988) J. Exp. Med. 168:2031). The authors of the North study concluded that "IL-1 only stimulates the replication of T cells that are already engaged in antitumor immune responses". It would follow then that if a tumor is non-immunogenic, IL-1$\beta$ would not be expected to affect the immune response against the tumor, since no immune responses are initiated by the tumor alone.

B16 murine melanoma is a rapidly growing metastatic tumor of spontaneous origin (Green (1977) Handbook of Genetically Standardized JAX Mice Second Ed., Bar Harbor Maine: Bar Harbor Times Publishing Co.). It has been widely studied (Ashley & Kotlarski (1986) Cell. Immunol. 101:157; Bystryn (1976) J. Immunology 116:1302; Leveson et al. (1979) Cancer Res. 39:582) and characterized as poorly or non-immunogenic tumor in syngeneic mice (Ashley & Kotlarski (1986) Cell. Immunol. 101:157; Bystryn (1976) J. Immunology 116:1302) and allogeneic mice (Ashley & Kotlarski (1986) Cell Immunol. 101:157). Tumors are characterized as poorly or weakly immunogenic if prior immunization with tumor cells fail to protect mice against subsequent challenge with the same tumor (Newitt et al. (1976) Br. J. Cancer 33:241). It has been reported in one system that B16 cells could protect mice from challenge with B16 cells (Celik et al. (1983) Cancer Res. 43:3507). In this system, B16 cells were placed in plastic diffusion chambers which were surgically implanted in the abdomen of mice. The B16 cells did not grow or metastasize from these chambers. 75% to 80% of the mice survived challenge with viable B16 cells when these diffusion chambers had been implanted for 4 to 8 weeks. The mechanism of the immunoprotection was not elucidated (Celik et al. (1983) Cancer Res. 43:3507). Without being confined to plastic diffusion chambers, B16 cells normally grow rapidly and kill the mice within 3 to 4 weeks (See Example 1).

## SUMMARY OF THE INVENTION

This invention relates to a method of using IL-1 either alone or in combination with cyto-reductive therapies such as surgery, radiation therapy or chemotherapy to enhance the immune response to weakly or non-immunogenic tumors.

Preferably, an immunogenic-enhancing amount of IL-1 is administered to a weakly or non-immunogenic tumor bearing mammal prior to conjuctive cyto-reductive therapy.

More preferably, Il-1 is administered to a weakly or non-immunogenic tumor bearing mammal less than ten consecutive days prior to conjunctive cyto-reductive therapy. Most preferred is the administration of IL-1, 5-7 consecutive days prior to surgical removal of such weakly or non-immunogenic tumor.

## DETAILED DESCRIPTION OF THE INVENTION

The consequences of in vivo injection of IL-1$\beta$ on the immune response to B16 cells has not been reported previously. Surgery, in conjunction with treatment with IL-1$\beta$, has allowed us to study these

consequences. We have determined that in vivo injections of IL-1β, in conjunction with cyto-reductive therapies, such as surgery, alters the immune response to B16 cells so that mice which have been treated with IL-1β and surgery, upon challenge with viable B16 cells, reject those B16 cells and do not grow tumors.

When IL-1β is injected into mice having B16 melanoma, daily for less than ten and preferably 5-7 consecutive days prior to surgical removal of the tumor, greater than 50% of the mice are cured of their tumors and reject subsequent challenge with viable B16 cells. In contrast less than 5% of the mice are cured and reject subsequent challenge with viable B16 cells when no IL-1 is given. These results show that IL-1β has antitumor properties and that IL-1β induces an effective immune response in mice against B16 melanoma, which is not detected in the absence of IL-1β treatment.

We have found that IL-1 can induce an effective and specific immune response, including a T-cell response, against a weakly immunogenic or non-immunogenic tumor, and thereby results, in conjunction with surgical removal of the tumor, in an increased likelihood of complete remission of the tumor. It is further expected that IL-1 treatment for about 5 to 7 days prior to surgical removal of a tumor will be useful in enhancing the production of tumor-infiltrating lymphocytes (TIL) from the tumor tissue for subsequent adoptive cellular immunotherapy for cancer. The production of TIL cells from surgically removed tumor tissue and their expansion and use in immunotherapy is described in the following references: Topalian et al. (1988) J. Clinical Oncology 6:839-853; Rosenberg et al. Immunotherapy of Patients with Metastatic Melanoma Using Tumor Infiltrating Lymphocytes and Interleukin-2: Preliminary Report,NCI, Bethesda, MD; Topalian et al. (1987) J. Immunol. Methods 102:127-141. An improved process for the ex vivo expansion of TIL is described in copending, coassigned patent application U.S.S.N. 07/351920, filed May 15, 1989.

In addition, it is expected that IL-1 treatment prior to surgical removal of a tumor will be useful as part of a tumor cell vaccine protocol. Tumor cell vaccine therapies involve the removal of tumor tissue and the subsequent administration of the tumor cells, which have been irradiated to stop continued growth, together with a nonspecific stimulator of the immune system, such as Bacillus Calmette-Guerin (BCG) (for review, see science (1989) 245:813-815). Based on our findings that IL-1 can induce a specific immune response to a tumor which is non-immunogenic in the absence of IL-1 treatment, it is expected that IL-1 treatment prior to surgery in a tumor cell vaccine protocol will increase the therapeutic efficacy of the tumor cell vaccine therapy.

In man, tumor-specific immune responses have not been detected, as determined by the presence of tumor-specific antibody, lymphocyte proliferation, or cellular cytotoxicity in peripheral blood cells (Vose et al. (1977) Int. J. Cancer 20:895; Klein et al. (1980) Contemporary Topics in Immunobiology 10:79-107; Vose and Moore (1979) Int. J. Cancer 24:579). These results suggest that many human tumors are relatively non-immunogenic. The treatment of cancer patients with IL-1 prior to surgery or other cyto-reductive therapy may decrease metastatic disease and tumor recurrence by promoting an immune response to non-immunogenic tumors.

Materials and Methods

Mice: Female C57BL/6 mice (7-12 weeks old, viral antibody free) were purchased from Charles River (Kingston, NY).

Tumors: B-16 cells, a murine melanoma, were obtained from National Cancer Institute (NCI) Tumor Repository, Frederick, MD and were negative for viruses by mense antibody production (MAP) testing and were mycoplasma free. B16 cells were maintained in vitro in RPM1 1640 (Gibco, Grand Island, NY) supplemented with 10% fetal calf serum (FCS) (Hyclone, Logan, VT). EL-4 cells, a murine T cell leukemia and P815 cells, a murine mastocytoma, were obtained from American Type Culture Collection and maintained in RPM1 1640 supplemented with 10% FLS. Cell lines were free of viruses and mycoplasma. MCA 105, a methylcholanthrene-induced murine sarcoma (Shu and Rosenberg (1985) Cancer Res. 45:1657) was obtained from the NCI from Dr. S. A. Rosenberg. MCA 105 cells were free of viruses and mycoplasma and were maintained in vitro in RMP1 1640 + 10% FCS.

IL-1β: The recombinant human (rH) and murine (rM) IL-1β were expressed in E. coli and purified by means of ion exchange and gel filtration chromatography (Huang et al. (1987) Mol. Biol. Med. 4:169; Huang et al. (1988) J. Immunology 140:3838). The biological activity, evaluated by a murine thymocyte assay (Lachman et al. (1985) Methods Enzymol. 116:467) was between 1 and $2x10^7$ units per mg of rH IL-1β and between 7 and $10x10^7$ units per mg of rM IL-1β. The endotoxin levels were 3-10 μg per mg of protein as determined by the QCL 1000 limulus amebocyte assay (MA Bioproducts, Walkerville, MD). IL-1β was diluted with D-PBS (Gibco) containing 0.5% normal C57BL/6 serum.

Tumor Therapy Experiments: B16 cells were resuspended in PBS at a cell concentration or $5x10^6$

cells/mL. Mice were injected intradermally (ID) on the ventral surface with 0.1 mL of B16 cell suspension. At this concentration, the tumors grew in 100% of the mice. Tumor weights (mg) were calculated by the formula: $0.5x(W^2xL^2)$, where W and L represent the width (mm) and length (mm) of the tumor. Mice with tumors weighing approximately 200 mg were used in these experiments. IL-1β (in 0.1 mL) was administered intramuscularly (IM) at 10 μg/mouse/day of rM IL-1β, or intratumorally (ITU) at 3 μg/mouse/day of rM IL-1β or 10 μg/mouse/day of rH IL-1β. Control mice were injected with PBS according to the same schedule as the IL-1β-treated mice. After one day following treatment with IL-1β or PBS, the mice were anesthetized with sodium pentabarbitol (50-60 mg/kg) and tumors were removed surgically. 60-90% of the mice survived surgery from both groups. Survival was monitored daily. Mice whose primary or secondary tumors grew to measure more than 2000 mg were sacrificed by cervical dislocation for humane reasons. The day at which they were sacrificed was included in the survival data.

Proliferation Assays: After 7 days of treatment with rH IL-1β (10 μg/mouse/day) or with PBS, mice were sacrificed by cervical dislocation and their spleen was removed. Spleens were disassociated into a single cell suspension using a "Stomacker" (Tekmar, Cincinnati, OH). The cell suspensions were washed 3x in PBS and counted with a hemocytometer and microscope. A differential cell count was done with Turk's stain and the spleen cells were adjusted to $2x10^6$ lymphocytes per mL. Spleen cells ($2x10^5$) were aliquoted into each well of a 96-well microtitre plate (Costar, Cambridge, MA). B16 and EL-4 cells were irradiated with a $^{137}$Cesium source (Cammacell 40, Atomic Energy of Canada Limited) for 130,000 and 22,000 rads, respectively, to inhibit their proliferation. Various concentration of B16 or EL-4 were added to the spleen cells in the microtitre plates. Each different concentration of tumor cells was assayed in triplicate. The cell mixtures were incubated for 2 days at 37° C in 5% $co_2$ in air. On the third day 1 μCi of $^3$H-thymidine (NEN, Boston, MA) was added to each well and incubated an additional 18 hours. The incorporation of $^3$H-thymidine into proliferating lymphocytes was measured by liquid scintillation. The spleen cells were harvested on paper discs by a cell harvester. The discs were dried then mixed with liquid scintillation fluid (NEN, Boston, MA) and counted on a beta liquid scintillation counter (Packard). The higher the counts per minute (CPM) of $^3$H-thymidine incorporated into the lymphocyte, the greater the immune response to the original tumor challenge. Spleen cells alone and tumor cells alone were also measured for their basal incorporation of $^3$H-thymidine. The incorporation of 3H-thymidine by tumor cells was subtracted from all cultures containing those tumor cells before the stimulation index (SI) was determined. (SI = S/C, where S is CPM of $^3$H-thymidine incorporated by lymphocytes stimulated by irradiated tumor cells and C is the CPM of $^3$H-thymidine incorporated by lymphocytes in the absence of tumor cells.) The larger the SI, the more vigorous the immune response to that tumor.

Mixed Lymphocyte Cultures: After 7 days of treatment with rH IL-1β (10 μ/mouse/day) or PBS, B16-bearing or normal mice were sacrificed by cervical dislocation and their spleens were removed. Spleens were disassociated into a single cell suspension using a Stomacker (Tekmar Co). The spleen cells were washed 3x in PBS and counted with a hemocytometer and microscope. Spleen cells were adjusted to $4x10^6$/mL and mixed with $4x10^5$/mL of irradiated B16 cells. The cell cultures were incubated for 3 days at 37° C in 5% $CO_2$ in air. On the fourth day, the cells were washed in PBS, recounted and adjusted to $4x10^6$/mL. The ability of these spleen cells to recognize and kill B16 cells or P815 cells was tested in a 4 hr $^{51}$Cr-release assay.

$^{51}$Cr-release Assay. The $^{51}$Cr-release assay described by Brunner et al. (Brunner et al. (1968) Immunology 14:181) was adapted for this study. P815 cells were labeled in suspension with 100 μCi of $Na_2{}^{51}CrO_4$ 1 ml (NEN, Boston) while B16 cells were labeled as adherent cells in a 96-well microtitre plate with 0.1 μCi of $Na_2{}^{51}CrO_4$/well. Cells were incubated with $Na_2{}^{51}CrO_4$ for 1 hr at 37° C then washed 3x in PBS. Spleen cells were added to $^{51}$Cr-labeled tumor cells (104) at 40:1, 20:1, 10:1 and 5:1 spleen cell to tumor cell ratios in 96-well microtitre plates (Costar). Each ratio was run in triplicate. After 4 hours, the plates were centrifuged and supernatants from each well were collected and counted in a gamma counter (LKB) . The amount of $^{51}$Cr-released into the supernatants is a measure of cell death. % cytotoxicity = (ER-SR)/(TR-SR), where ER is release of $^{51}$Cr from target cells killed by lymphocytes, SR is the spontaneous release of $^{51}$Cr from target cells incubated with medium alone, and TR is the total release of $^{51}$Cr from target cell lysed by a detergent, Triton x-100.

Example 1

IL-1α and IL-1β have been reported to fail to cause complete regression, i.e., fail to "cure", transplanted B16 tumors in mice (Nakamura et al. (1986) GANN 77:767; Nakata et al. (1988) Cancer Res. 48:584). Because B16 tumors are rapidly growing and metastatic tumors, failure to completely inhibit the growth of the primary tumor results in death of the mice within a very short time period (see Table 1). The data

presented in Table 1 represent an experiment in which the tumor-bearing mice were given 7 daily injections of IL-1$\beta$ intratumorally (ITU), these injections resulted in 90% Inhibition of the growth of the tumor and increased the median survival time of the mice from 21 to 34 days. However, all the mice treated with IL-1$\beta$ were dead by 42 days.

Table 1

Treatment with IL-1β Alone Reduces the Size of B16 Tumors and Increases the Median Survival of Mice with B16 Tumors But Does Not "Cure" Them

| Treatment* | % inhibition of of tumor growth+ | Day of 50% survival++ |
|---|---|---|
| PBS | --- | 21 (34) |
| IL-1β | 90% | 34 (42) |

* C57BL/6 mice (N=13/group) were injected daily with PBS or rH IL-1β (10 μg/mouse/day) for seven consecutive days from days 10-17 after ID implantation of $5x10^5$ B16 cells.

\+ On day 18, tumors were measured and weights calculated; % inhibition of tumor growth = 1 - T/Cx100% where T

Table 1 (continued)

is the mean tumor weight of mice who received IL-1β and C is the mean tumor weight of mice who received PBS.

++ The day on which 50% of the mice remained alive is given; the number in parenthesis is the day on which the last mouse in each group died.

These data indicate that IL-1$\beta$, by itself, does not cure mice of B16 tumors in this model.

Example 2

The shortcomings of the model reflected in Table 1 are overcome by the model we used in the present invention. In this model we can examine the effects of IL-1$\beta$ on the immune response to B16 tumors in a manner which more closely mimics human solid tumors. In man, the surgical removal of primary solid tumors is not a major problem with the exception of primary brain and liver tumors. The eventual spread of these tumors after surgery is the major cause of death in cancer patients. In the present B16 melanoma model, we treat mice with IL-1$\beta$ before surgery then monitor the effects of IL-1$\beta$ on metastatic spread. If no metastases occur, then we artificially simulate metastatic spread by injecting a second challenge of B16 cells. When this model was used, the following results were obtained (see Table 2).

Table 2

IL-1β Promotes an Immune Response Against the Non-immunogenic B16 Melanoma

| Treatment+ | Day of 50% survival++ | % survival on day 120 | % survival of mice given second B16 challenge* | % survival of total mice on day 240 |
|---|---|---|---|---|
| PBS | 21 | 0 (0/13) | 0 | 0 |
| PBS + surgery | 48 | 21 (5/24) | 20 (1/5) | 4 (1/24) |
| IL-1β + surgery | 240 | 65 (15/23) | 80 (12/15) | 52 (12/23) |

These results are a composite of four different experiments using the same protocol.

\+ Mice were injected ID with $5 \times 10^5$ B16 on day 0. On day 10-17 mice were injected with either PBS ITU or rM IL-1β ITU (3 μg/mouse/day) or IM (10 μg/mouse/day). On day 19, one PBS group and the IL-1β group had their tumors surgically removed. One PBS group had no surgery. Survival was monitored.

++ The day of which 50% of the mice are alive.

* On day 120, mice are injected ID with $10^5$ B16 cells.

As seen in Table 2, only 21% (5/24) of the mice in the surgery alone group survived until day 120. When these 5 mice were re-challenged with B16 cells, only 20% (1/5) of the mice survived until day 240. Thus, only 4% (1/24) of the mice who received surgery alone survived 240 days following B16 implant and rechallenge. In contrast, 65% (15/23) of the mice treated with IL-1β plus surgery survived up to 120 days. When these 15 mice were re-challenged with B16 cells 80% (12/15) survived at day 240. Thus, a total of

52% of the mice in the IL-1β plus surgery group survived 240 days.

Example 3

Seven of the mice in the IL-1β plus surgery group, who rejected the second challenge of B16 cells, were injected with another syngeneic tumor, MCA 105 (Shu and Rosenberg (1985) Cancer Res. 45:1657). 100% (7/7) of these mice grew MCA 105 (See Table 3) . Thus, the immune response to B16 promoted by IL-1β treatment is specific to B16 and provided no protection against the unrelated tumor MCA 105.

Table 3

IL-1β Specifically Promotes an Immune Response to B16 Melanomas

| Treatment+ | % survival after second challenge with B16 cells++ | % survival after challenge with MCA 105* |
|---|---|---|
| IL-1β | 100% (7/7) | 0% (0/7) |

Table 3 (continued)

\+ 7 C57BL/6 mice were transplanted with B16 cells and received 7 daily injections of IL-1β ITU (rM IL-1β; 3 μg/mouse/day), then had their primary B16 removed surgically.

++ These mice survived 120 days then received a second challenge of B16 cells on day 120 and survived another 60 days without any sign of tumor.

* On day 180 these mice were injected ID with $10^5$ MCA 105 tumor cells. Survival at day 240 was monitored.

These results suggest that injections of IL-1β prior to surgery inhibit metastatic spread of B16 and that inhibition of metastatic spread may be due to the ability of IL-1β to specifically alter immune responses to B16 melanoma in vivo prior to surgery.

Example 4

Since so few mice 21% (5/24) in the group treated by surgery alone survived to 120 days (Table 2), we removed the B16 tumors sooner so that more mice would survive until 120 days (See Table 4). In Table 4, mice were injected ID with $5x10^5$ B16 cells, and 10 days later the primary tumors were surgically removed from one group of mice (N = 11). In a second group (N = 8), IL-1$\beta$ was injected daily ITU or ID for 5 consecutive days starting on day 10. On day 17 this group of mice had their tumors surgically removed. Survival was monitored for both groups. The results of this study are shown in Table 4.

Table 4

IL-1β Promotes Immunity to B16 Melanomas

| Treatment+ | % Survival on day 120 | % of mice with tumor after second challenge with B16++ |
|---|---|---|
| Surgery | 73 (8/11) | 100 (8/8) |
| Surgery + IL-1β | 100 (8/8) | 37 (3/8) |

\+ C57BL/6 mice were injected with $5x10^5$ B16 cells on day 0. On day 10, tumors were removed from 15 mice and 10 mice received rM IL-1β (3 μg/mouse/day) ITU for 5 consecutive days. On day 17 these mice had their tumor removed surgically.

++ On day 120, mice were injected with $10^5$ B16 cells and growth of tumor was monitored.

As shown in Table 4, 73% (8/11) of the mice in the surgery alone group survived to 120 days because the tumors were excised early (10 days after implantation). By comparison 100% (8/8) of the mice who received IL-1$\beta$ plus surgery survived to 120 days. When the mice in the surgery alone group were re-challenged with B16 cells, 100% (8/8) of the mice grew tumors and 50% (4/8) died within 21 days after second challenge. Only 37% (3/8) of the mice in the IL-1$\beta$ plus surgery group grew tumors after a second challenge with B16 and the 3 that grew tumors remained alive at 21 days after the second challenge. These results support the results in Table 2. Secondly these results suggest that IL-1$\beta$ inhibits the metastatic spread of B16 by altering the host immune response to B16 tumors, rather than by simply reducing the size of tumor (which does occur when IL-1$\beta$ is given; Table 1).

Example 5

If IL-1$\beta$ changes the ability of the immune system to recognize a weakly immunogenic tumor like B16

(Ashley & Kotlarski (1986) Cell. Immunol. 101:157; Bystryn (1976) J. Immunology 116:1302; Leveson et al. (1979) cancer Res. 39:582), then in vitro detection of this specific immunity against B16 cells should be apparent in IL-1 treated B16-bearing mice, but not in non-treated (PBS-treated) mice. When spleen cells from C57BL/6 mice bearing B16 tumor were injected with IL-1β for 5-7 consecutive days, specific immune response against B16 cells was evident by lymphocyte proliferation assays (Table 5) and by specific lymphocyte cytotoxicity (Table 6).

Table 5

Injections of IL-1β into B16-bearing Mice Promotes Specific T Cell and B Cell Responses to B16 Melanoma Cells

| Treatment+ | S/T Ratio++ | $^3$H-thymidine incorporation (CPM±SD)* EL-4 | B16 |
|---|---|---|---|
| PBS | 40:4 | 1488±407(3.5)** | 60±170(0.1) |
| | 40:2 | 1074±161(2.3)** | 532±79(1.0) |
| | 40:1 | 254±257(0.6) | 189±349(0.4) |
| | None | 431±48 | 506±63 |
| IL-1β | 40:4 | 3245±706(1.2) | 12193±1646(4.3)** |
| | 40:2 | 3400±1271(1.3) | 5617±2396(2.0)** |
| | 40:1 | 3410±1079(1.3) | 4149±869(1.5) |
| | None | 2675±506 | 2823±349 |

\+ C57BL/6 mice were transplanted with B16 cells on day 0. On days 10 - 17 they received either PBS or IL-1 (10 μg/mouse/day) ITU. On day 18, the mice were sacrificed and spleen harvested.

++ Irradiated EL-4 or B16 cells were added to $2x10^5$ spleen cells at the stated spleen cell-to-tumor cell (S/T) ratio and incubated for 3 days.

Table 5 (continued)

* On day 2, $^{3}$H-thymidine was added to each culture; on day 3 the cells were harvested and the amount (CPM) of $^{3}$H-thymidine incorporated into lymphocytes measured by liquid scintillation counting. The numbers in parentheses are the stimulation index: SI = T/M, where T is mean CPM of $^{3}$H-thymidine incorporated by spleen cells mixed with tumor cells divided by mean CPM of $^{3}$H-thymidine incorporated by spleen cells incubated with no tumor cells.

** Denotes significant ($p \geq 0.05$) spleen lymphocyte proliferative responses to tumors.

In contrast, without treatment with IL-1$\beta$, no immune responses were evident in spleen cello of B16-bearing mice (see Tables 5 and 6). In Table 5, spleen cells from 5 mice from each group, PBS- or IL-1-treated, were pooled and mixed with either irradiated B16 cells or EL-4 cells (known antigenic cells) and incubated 3 days in culture. The lymphocyte response to the tumor cells was measured by the incorporation of $^{3}$H-thymidine. Spleen lymphocyte cells from the IL-1-treated group of mice, specifically proliferated in response to B16 cells but not to EL-4 cells which are antigenic.

The spleen cells from mice treated with PBS did not respond to B16 cells but did respond to the antigenic tumor EL-4 (Table 5). The lack of response to EL-4 cells in B16-bearing mice treated with IL-1 demonstrates the specificity of the IL-1-mediated response and may result from the immunological phenomena of "antigen competition" (Hokama and Nakamura (1982) Immunology and Immunopathology, Little Brown, Boston, MA, pp. 115-116). In this phenomena a "dominant" antigen inhibits the response to another antigen. In the case of IL-1-treated, B16-bearing mice, B16 cells appear to represent the dominant antigen which suppressed the response to the immunogenic EL-4 cell. The PBS-treated mice did not recognize the B16 cells as a "dominant" antigen and no antigenic competition occurred when the spleen cells were mixed with the antigenic tumor EL-4 (Table 5) . These results show that a specific immune response to B16 cells, as measured by antigen-induced lymphocyte proliferation, occurs in IL-1$\beta$-treated mice but not in PBS-treated mice. These results suggest that IL-1$\beta$ promotes the ability of the immune system to recognize implanted B16 tumor cells.

Example 6

Besides a lymphocyte proliferative response to B16 cells, a specific in vitro cytotoxic response was generated from spleen cells from IL-1$\beta$-treated B16-bearing mice (Table 6). To demonstrate this normal mice or mice bearing B16 tumors were injected with PBS or rH IL-1$\beta$ ITU for 7 consecutive days. On the eighth day the mice were sacrificed and spleens were removed. Five spleens from each group were pooled. Spleen cells were mixed with irradiated B16 cells at a cell ratio of 10:1 lymphocytes to tumor cell, and incubated for 3 days. On the 4th day, the spleen cells were tested for their ability to kill B16 cells or P815 cells using a 4 hour $^{51}$Cr-release assay. Only spleen cells from B16-bearing mice who received IL-1$\beta$ killed B16 cells (Table 6). Spleen cells from mice who received IL-1$\beta$, but did not have B16 tumors, did not kill B16 cells. Also, spleen cells from mice who had B16 tumors, but did not receive IL-1$\beta$, did not kill B16 cells. These results indicate that injections of IL-1$\beta$ provokes a specific immune response against B16 cells

which is not detected in the absence of IL-1β or in the absence of B16 tumors. Both IL-1β and B16 tumor must be present to detect specific T cell mediated cytotoxicity.

Table 6

Injections of IL-1β into B16-bearing Mice Promotes Specific T Cell Killing of B16 Cells

| Treatment* | B16-bearing** | $LU_{30}/10^7$ spleen cells*** | |
|---|---|---|---|
| | | P815 | B16 |
| PBS | No | 0 | 0 |
| PBS | Yes | 0 | 0 |
| IL-1β | No | 0 | 0 |
| IL-1β | Yes | 0 | 38 |

* Mice were injected daily ITU or ID with PBS or rH IL-1β ITU (10 μg/mouse/day) or ID (30 μg/mouse/day) for 7 consecutive days.

** B16-bearing mice were prepared by injection with $5x10^5$ B16 cells ID. Treatment of mice with PBS or IL-1β was started on day 10 after implantation of B16 cells.

*** Spleen cells were incubated *in vitro* for 3 days with irradiated B16 cells at a 10:1 spleen cell to B16 cell ratio. On day 4 spleen cells were assayed for their ability to kill either P815 or B16 cells in a 4 hour $^{51}Cr$-release assay at 4 different spleen cell to target cell ratios. If killing occurred, the number of spleen cells that killed 30% of the target cells ($LU_{30}$) was determined by linear regression analysis (30). The higher the number the more cytotoxic are the spleen cells.

## Dosage and Administration

IL-1 can be administered as a sterile, nonpyrogenic parenteral solution. The aqueous parenteral vehicle could be, for example, Sterile Water for Injection USP, 0.9% Sodium Chloride for Injection USP or 5%

Dextrose Injection USP. Pharmaceutical compositions of the present invention comprise an effective amount of purified IL-1β and a pharmaceutically acceptable carrier such as listed above, and optionally additional excipients, such as preservatives and buffers as known to those skilled in the art of pharmaceutical formulations. Sec, for example, Remington's Pharmaceutical Sciences, 17th Edition, A. Osol (1985), a standard reference in the field. IL-1 may be prepared in a stable formulation ready for administration, or for dilution in an appropriate intravenous solution. Preferably, to increase product shelf life, IL-1 may be formulated, for example, as a sterile lyophilized powder to be reconstituted aseptically as a buffered solution.

As is known to those skilled in the art, individual patient dosages will vary depending upon known factors such as the pharmacodynamic characteristics of the particular active ingredient, and its mode and the route of administration; the age, health, and weight of the recipient; the nature and extent of symptoms; the kind of concurrent treatment and frequency of treatment; and the effect desired. In practicing the present invention, purified IL-1 is administered to a mammal at a therapeutically effective dosage level. Injection either intratumorally, intraarticularly, intramuscularly, intravenously or intraperitoneally is the most practical method of administration.

## Claims

1. A method of enhancing the immunogenic response to a weakly immunogenic or non-immunogenic tumor comprising administering to a mammal having a weakly or non-immunogenic tumor an immunogenic enhancing amount of IL-1 prior to conjunctive cyto-reductive therapy of the tumor.

2. A method of Claim 1 wherein the enhanced immunogenic response is a specific immune response.

3. A method of Claim 1 wherein the immunogenic response includes a T-cell mediated immune response.

4. A method of Claim 3 wherein the T-cell immune response is specific for the weakly or non-immunogenic tumor.

5. A method of Claim 1 wherein the IL-1 is administered periodically during a period of less than 10 consecutive days prior to cyto-reductive therapy.

6. A method of Claim 4 wherein the IL-1 is administered daily for 5-7 consecutive days prior to cyto-reductive therapy.

7. A method of Claim 5 wherein the IL-1 is IL-1β.

8. A method of Claim 6 wherein the IL-1β is human.

9. A method of Claim 6 wherein the IL-1β is recombinant.

10. A method of Claim 1 wherein the conjunctive cyto-reductive therapy is surgical removal of all or part of the weakly or non-immunogenic tumor.

11. A method of Claim 2 wherein the conjunctive cyto-reductive therapy is surgical removal of all or part of the weakly or non-immunogenic tumor.

12. A method of Claim 3 wherein the conjunctive cyto-reductive therapy is surgical removal of all or part of the weakly or non-immunogenic tumor.

13. A method of Claim 4 wherein the conjunctive cyto-reductive therapy is surgical removal of all or part of the weakly or non-immunogenic tumor.

14. A method of Claim 5 wherein the conjunctive cyto-reductive therapy is surgical removal of all or part of the weakly or non-immunogenic tumor.

15. A method of Claim 6 wherein the conjunctive cyto-reductive therapy is surgical removal of all or part of the weakly or non-immunogenic tumor.

**16.** A method of Claim 7 wherein the conjunctive cyto-reductive therapy is surgical removal of all or part of the weakly or non-immunogenic tumor.

**17.** A method of, Claim 8 wherein the conjunctive cyto-reductive therapy is surgical removal of all or part of the weakly or non-immunogenic tumor.

**18.** A method of Claim 9 wherein the conjunctive cyto-reductive therapy is surgical removal of all or part of the weakly or non-immunogenic tumor.

**19.** A method of reducing the recurrence of a surgically removed weakly or non-immunogenic tumor comprising administering to a mammal having a weakly or non-immunogenic tumor an effective amount of IL-1β 5-7 days prior to surgically removing all or part of such tumor.

**20.** A method of enhancing expansion of tumor-infiltrating lymphocytes ex vivo comprising administering to a mammal with a tumor, prior to surgical removal of all or part of such tumor, an effective amount of IL-1.

**21.** A method of Claim 18 wherein the IL-1 is IL-1β.

**22.** A method of Claim 19 wherein the IL-1β is human.

**23.** A method o Claim 20 wherein the IL-1β is recombinant.

European Patent Office

# PARTIAL EUROPEAN SEARCH REPORT

which under Rule 45 of the European Patent Convention shall be considered, for the purposes of subsequent proceedings, as the European search report

Application number

EP 91103057.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | No documents have been disclosed<br>---- | | A 61 K 39/39<br>A 61 K 45/05 |
| | | | TECHNICAL FIELDS SEARCHED (Int. Cl.5) |

## INCOMPLETE SEARCH

The Search Division considers that the present European patent application does not comply with the provisions of the European Patent Convention to such an extent that it is not possible to carry out a meaningful search into the state of the art on the basis of ~~some~~ all of the claims.

Claims searched completely:

Claims searched incompletely:

Claims not searched:

Reason ~~for the limitation of the search~~:

Method of treatment of the human or animal body therapy. Article 52(4) EPC.

| Place of search | Date of completion of the search | Examiner |
|---|---|---|
| VIENNA | 04-06-1991 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document